# EUROPEAN PATENT APPLICATION

(11) **EP 2 910 269 A1**
(43) Date of publication of application: **26.08.2015**
(21) Application number: 14726064.0
(22) Date of filing: 07.01.2014
(51) Int. Cl.: A61M 11/02

(54) **ATOMIZER WITH MULTIPLE CYLINDER BLOCKS SEPARATING AIR INLET AND AIR OUTLET**

(30) Priority: 18.11.2013 CN 201320732186 U
(71) Applicant: LEE, Ming-hsien, Nantun Dist. Taichung City, Taiwan (CN)
(72) Inventor: LEE, Wen-ching, Taichung City Taiwan (CN)
(74) Representative: Lang, Christian
(86) International application number: PCT/CN2014/070202
(87) International publication number: WO 2015/070527

(57) **Abstract**

The present invention relates to an atomizer using a plurality of cylinder units to separate an inlet from an outlet. The atomizer includes first and second cylinder units therein. The first cylinder unit includes a first intake port. The second cylinder unit includes a second outtake port. The first intake port and the second outtake port are respectively connected to an inlet and an outlet of the atomizer by connection pipes. Since the inlet and the outlet of the atomizer are independent from and not in communication with each other, cross infection will not occur, providing use safety.

## Description

### TECHNICAL FIELD

The present invention relates to an atomizer and, more particularly, to an atomizer using a plurality of cylinder units to separate an inlet from an outlet.

### BACKGROUND OF THE INVENTION

An atomizer generally includes a main body having an inlet or outlet for sucking, injecting, or atomization purposes. The atomizer can be used in medical treatments, such as medical care of nasal cavities and throats of patients in ear-nose-throat departments of hospitals and elderly healthcare centers. In an application of the sucking function, a nasal mucus sucker can be used with the main body of the atomizer to suck nasal mucus and accumulated phlegm out of the nasal cavity of a patient. In an application of the injecting function, an injector (or nasal spray) can be used with the main body to clean the nasal cavity, the throat or a wound of a patient. In another application of the injecting function, a sprayer can be used with the main body to proceed with spray treatment in the nasal cavity or throat.

In an arrangement, the main body of the atomizer includes a housing receiving a motor and a cylinder unit having an inlet and an outlet. A check valve is mounted in each of an inlet passage for the inlet and an outlet passage for the outlet of the cylinder unit, providing the cylinder unit with an intake function and an outtake function. Each of the inlet and the outlet is connected by a connection pipe to an exterior of the housing. Thus, the cylinder unit includes an inlet piping and an outlet piping. An outlet end of the inlet piping forms the inlet of the atomizer, and the outlet end of the outlet piping forms the outlet of the atomizer. The inlet piping is not in communication with the outlet piping under normal conditions. However, the check valves are generally flexible resilient members in which the inlet piping or the outlet piping is opened or closed upon flexing or not flexing of the resilient members. Elastic fatigue of the check valves could occur after the atomizer has been used over a period of time. Thus, incomplete closing could occur though the inlet piping or the outlet piping should be closed, leading to undesired communication between the inlet piping and the outlet piping and causing risks of cross infection. Thus, the present inventor deems improvement is necessary.

### BRIEF SUMMARY OF THE INVENTION

To solve the defects of the current techniques, an objective of the present invention is to provide an atomizer using a plurality of cylinder units to separate an inlet from an outlet to assure that a first intake piping will not be in communication with a second outtake piping. Even if elastic fatigue of the intake check valve and the outtake check valve occurs after they have been used over a period of time, the first intake piping will not be in communication with the second outtake piping. Thus, cross infection during use can be avoided more effectively. A nasal mucus sucker, injector (or nasal spray), sprayer, or breast pump can be connected to the inlet or the outlet. Users can feel ease during use.

The primary technical solution of the present invention is an atomizer including:
a housing having an inlet and an outlet;
a motor mounted in the housing and including an eccentric transmission shaft;
a first cylinder unit mounted in the housing, with the first cylinder unit including a piston and a coupling bearing connected to the piston, with the eccentric transmission shaft extending through the coupling bearing, with the piston moving reciprocatingly when the motor operates, with the first cylinder unit further including a first intake port, with an intake check valve mounted in a first intake passage for the first intake port of the first cylinder unit, with the first intake port of the first cylinder unit connected to an end of a connection pipe, with the other end of the connection pipe connected to the inlet, forming a first intake piping; and
a second cylinder unit mounted in the housing, with the second cylinder unit including a piston and a coupling bearing connected to the piston, with the eccentric transmission shaft extending through the coupling bearing, with the piston moving reciprocatingly when the motor operates, with the second cylinder unit further including a second outtake port, with an outlet check valve mounted in a second outtake passage for the second outtake port, with the second outtake port connected to an end of a connection pipe, with the other end of the connection pipe connected to the outlet, forming a second outtake piping.

The advantageous effects of the present invention are that the first cylinder unit of the present invention includes the first intake piping and the second cylinder unit includes the second outtake piping. Furthermore, the other end of the connection pipe for the first intake piping and the other end of the connection pipe for the second outtake piping are respectively connected to the inlet and the outlet. This assures that the first intake piping will not be in communication with the second outtake piping. Even if elastic fatigue of the intake check valve and the outtake check valve occurs after they have been used over a period of time, the first intake piping will not be in communication with the second outtake piping. Thus, cross infection during use can be avoided more effectively. A nasal mucus sucker, injector (or nasal spray), sprayer, or breast pump can be connected to the inlet or the outlet. Users of these devices can feel ease during use. The term "a plurality of" used herein means at least two.

### DESCRIPTION OF THE DRAWINGS

FIG. 1 is a perspective view of the present invention.
FIG. 2 is a top view of the present invention, with some components of the atomizer shown in phantom lines.
FIG. 3 is a perspective view of a dual cylinder structure of the present invention.
FIG. 4 is a perspective view of the dual cylinder structure of the present invention.
FIG. 5 is a side view of the dual cylinder structure of the present invention, illustrating movement of pistons.
FIG. 6 is another side view of the dual cylinder structure of the present invention, illustrating movement of the pistons.
FIG. 7 is an exploded, perspective view of a portion of a cylinder unit of the present invention.
FIG. 8 is a cross sectional view of the cylinder unit of the present invention.

### Number list

- A: atomizer
- 1: housing
- 11: inlet
- 12: outlet
- 13: first vent
- 14: second vent
- 2: motor
- 21: eccentric transmission shaft
- 3: first cylinder unit
- 31: cylinder body
- 311: top wall
- 312: inlet opening
- 313: first rib
- 314: first spacing
- 315: outlet opening
- 316: second rib
- 317: second spacing
- 32: cover
- 321: partitioning wall
- 322: inlet section
- 323: outlet section
- 324: hollow tube
- 325: first intake port
- 326: first outtake port
- 33: spacer plate
- 331: punched hole
- 332: intake check valve
- 333: outtake check valve
- 34: piston
- 35: coupling bearing
- 36: connection pipe
- 36A: connection pipe
- 4: second cylinder unit
- 41: cylinder body
- 42: cover
- 421: second intake port
- 422: second outtake port
- 43: piston
- 44: coupling bearing
- 45: connection pipe
- 45A: connection pipe
- 5: mounting frame

### DETAILED DESCRIPTION OF THE INVENTION

The structure, features, and embodiments of the present invention will be described in connection with the accompanying drawings such that the Examiner will have a better understanding of the present invention.

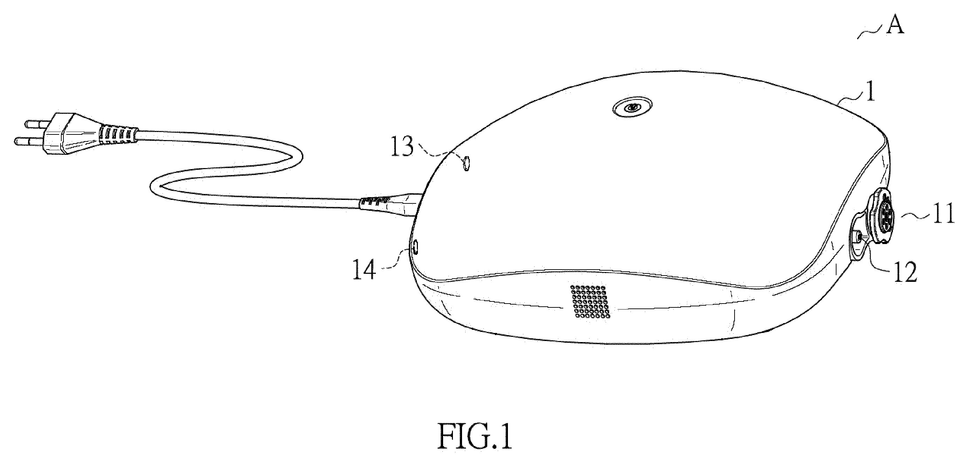

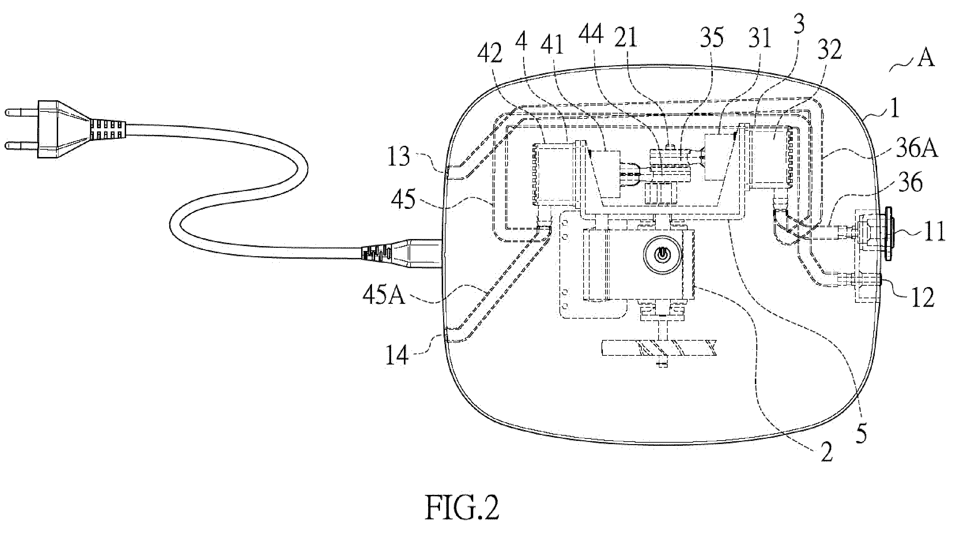

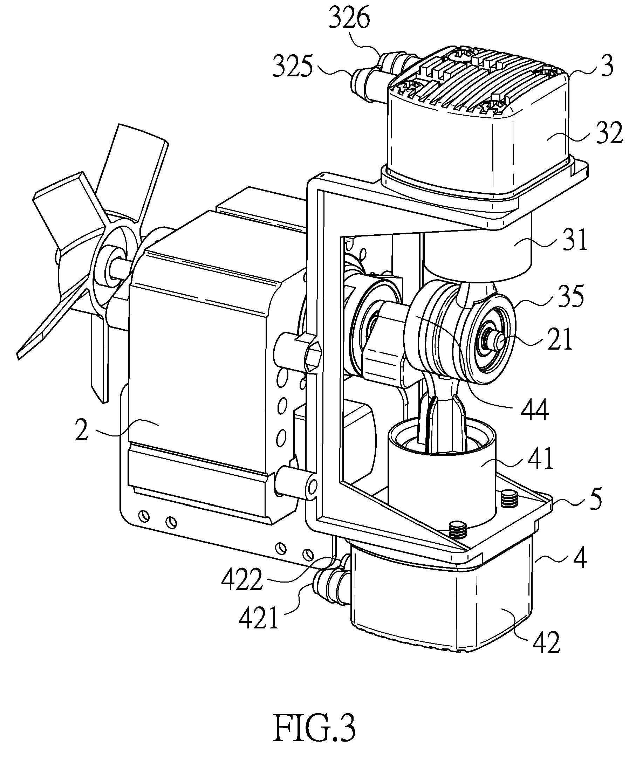

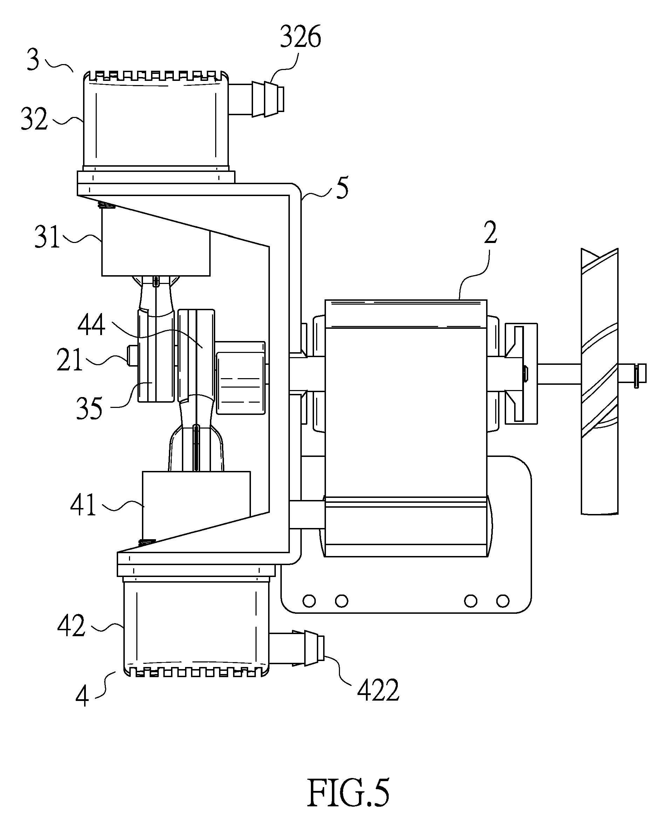

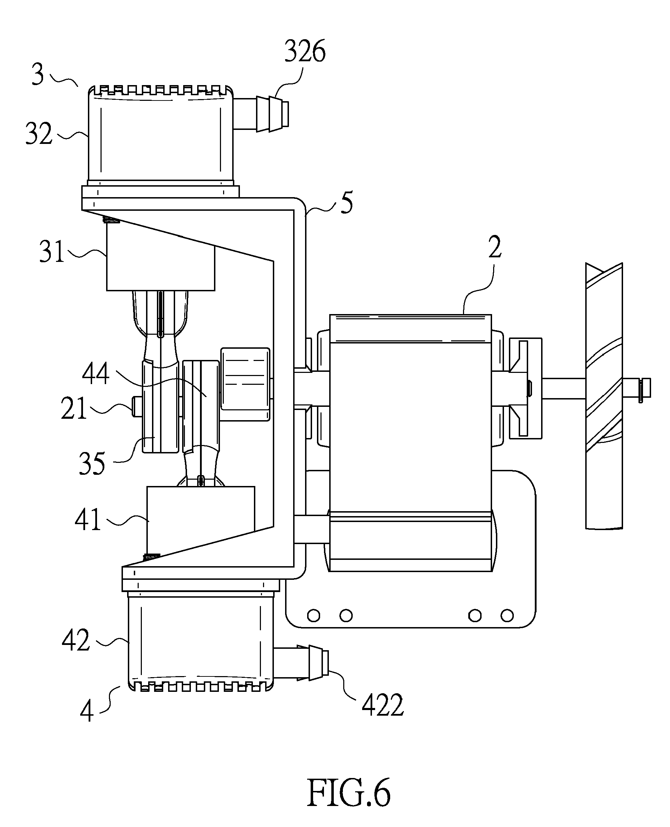

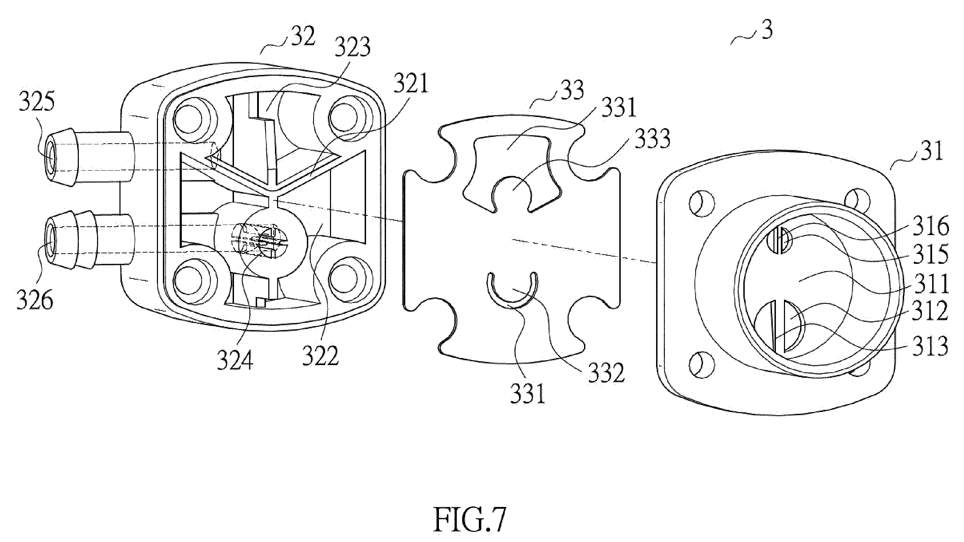

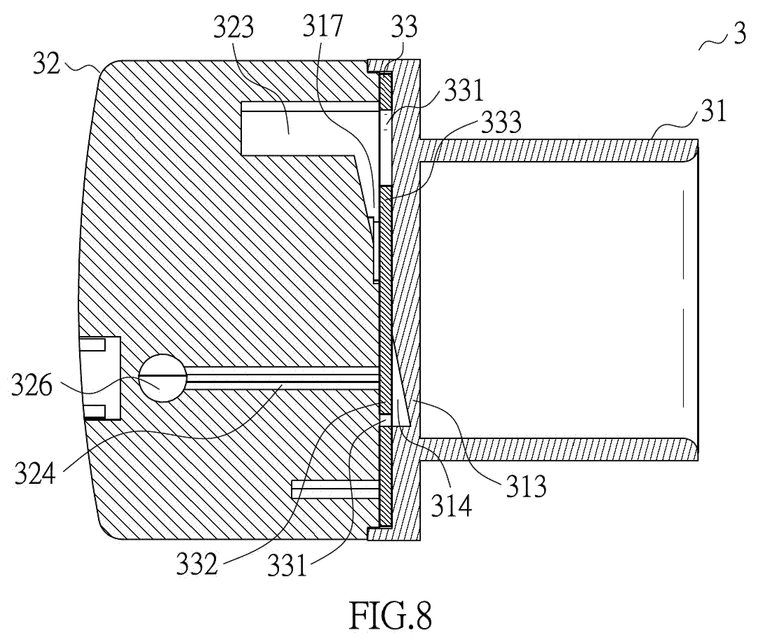

With reference to FIGS. 1-8, the present invention is an atomizer using a plurality of cylinder units to separate an inlet from an outlet. The atomizer A includes a housing 1 having an inlet 11 and an outlet 12.

A motor 2 is mounted in the housing 1 and includes an eccentric transmission shaft 21.

A first cylinder unit 3 is mounted in the housing 1 and includes a piston 34 and a coupling bearing 35 connected to the piston 34. The eccentric transmission shaft 21 extends through the coupling bearing 35. The piston 34 moves reciprocatingly when the motor 2 operates. The first cylinder unit 3 further includes a first intake port 325. An intake check valve 332 is mounted in a first intake passage for the first intake port 325. The first intake port 325 is connected to an end of a connection pipe 36. The other end of the connection pipe 36 is connected to the inlet 11, forming a first intake piping.

A second cylinder unit 4 is mounted in the housing 1 and includes a piston 43 and a coupling bearing 44 connected to the piston 43. The eccentric transmission shaft 21 extends through the coupling bearing 44. The piston 43 moves reciprocatingly when the motor 2 operates. The second cylinder unit 4 further includes a second outtake port 422. An outlet check valve (not shown) is mounted in a second outtake passage for the second outtake port 422. The second outtake port 422 is connected to an end of a connection pipe 45. The other end of the connection pipe 45 is connected to the outlet 12, forming a second outtake piping. Thus, the inlet 11 and the outlet 12 are respectively in communication with the first and second cylinder units 3 and 4 different from each other, avoiding cross infection. In the preferred embodiment, the motor 2 further includes a mounting frame 5 fixed to a side thereof. The first and second cylinder units 3 and 4 are mounted to the mounting frame 5, with the eccentric transmission shaft 21 located between the first and second cylinder units 3 and 4. The first and second cylinder units 3 and 4 are spaced from each other by 180 degrees as viewed from a longitudinal axis of the eccentric transmission shaft 21.

The first cylinder unit 3 of the present invention includes the first intake piping and the second cylinder unit 4 includes the second outtake piping. Furthermore, the other end of the connection pipe 36 for the first intake piping and the other end of the connection pipe 45 for the second outtake piping are respectively connected to the inlet 11 and the outlet 12. This assures that the first intake piping will not be in communication with the second outtake piping. Even if elastic fatigue of the intake check valve 332 and the outtake check valve (not shown) occurs after they have been used over a period of time, the first intake piping will not be in communication with the second outtake piping. Thus, cross infection during use can be avoided more effectively. A nasal mucus sucker, injector (or nasal spray), sprayer, or breast pump can be connected to the inlet 11 or the outlet 12. Users can feel ease during use. Furthermore, since the first and second cylinders 3 and 4 are paced from each other by 180 degrees as viewed from the longitudinal axis of the eccentric transmission shaft 21, vibrations resulting from reciprocating movements of the cylinders 34 and 43 are counterbalanced by each other. Thus, the atomizer A operates more smoothly than atomizers having only one cylinder unit. The term "a plurality of" used herein means at least two.

In this embodiment, the first cylinder unit 3 further includes a first outtake port 326. An outtake check valve 333 is mounted in a first outtake passage for the first outtake port 326. The first outtake port 326 is connected to an end of a connection pipe 36A. The other end of the connection pipe 36A is connected to a first vent 13 of the atomizer A, forming a first outtake piping. The second cylinder unit 4 further includes a second intake port 421. An inlet check valve (not shown) is mounted in a second intake passage for the second intake port 421. The second intake port 421 is connected to an end of a connection pipe 45A. The other end of the connection pipe 45A is connected to a second vent 14 of the atomizer A, forming a second intake piping. The first and second vents 13 and 14 allow more smooth operations of the first and second cylinder units 3 and 4. Furthermore, the first and second vents 13 and 14 will not be connected to the nasal mucus sucker, injector (or nasal spray), sprayer, or breast pump. Thus, the present invention can be used with ease without the risk of cross infection.

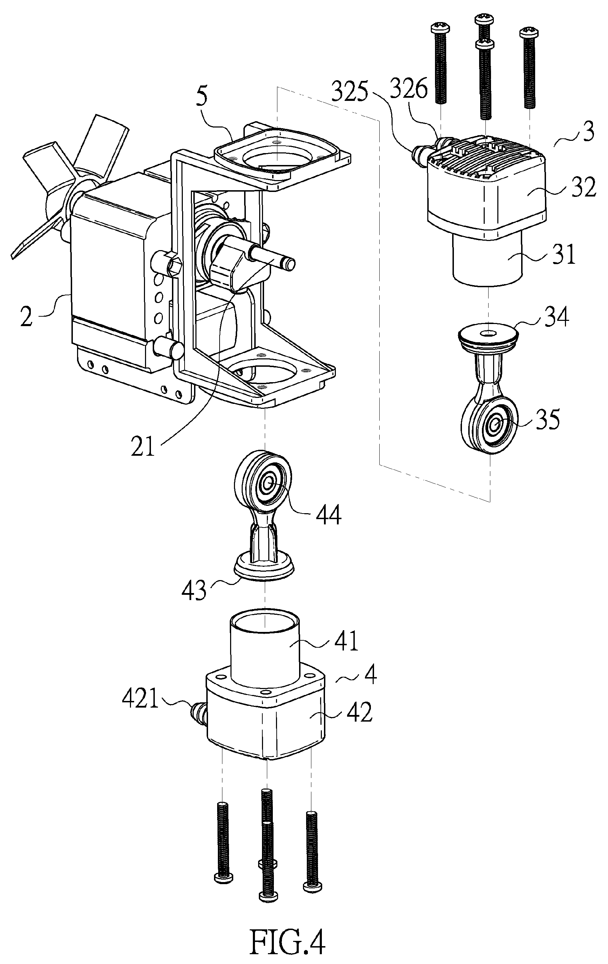

With reference to FIGS. 4, 7, and 8, there are many cylinder units including the first intake port 325, the first outtake port 326, the second intake port 421, and the second outtake port 422. The cylinder units should not be limited to the following embodiment. In the embodiment, each of the first and second cylinder units 3 and 4 includes a cylinder body 31, 41 and a cover 32, 42 covering the cylinder body 31, 41. The first intake port 325 and the first outtake port 326 are located in the cover 32 of the first cylinder unit 3. The second intake port 421 and the second outtake port 422 are located in the cover 42 of the second cylinder unit 4. The first and second cylinder units 3 and 4 are substantially identical to each other. Thus, the first cylinder unit 3 is used as an example, wherein the first cylinder unit 3 includes a spacer plate 33 between the cylinder body 31 and the cover 32. A top wall 311 is provided on a side of the cylinder body 31 facing the cover 32. The top wall 311 includes an inlet opening 312 and an outlet opening 315. The cover 32 includes an upper wall. A partitioning wall 321 extends from the upper wall of the cover 32 toward the cylinder body 31. A bottom face of the partitioning wall 321 abuts the spacer plate 33. The upper wall of the cover 32, the partitioning wall 321, and the spacer plate 33 divide an interior of the cover 32 into an inlet section 322 and an outlet section 323 not in communication with the inlet section 322. The inlet opening 312 is in communication with the inlet section 322 and the first intake port 325 to form the first intake passage. The outlet opening 315 is in communication with the outlet section 323 and the first outtake port 326 to form the first outtake passage. The spacer plate 33 includes two punched holes 331 respectively aligned with the inlet opening 312 and the outlet opening 315. Remaining materials in the punched holes 331 are connected to the spacer plate 33 and respectively form the intake check valve 332 and the outtake check valve 333. The cover 32 includes a hollow tube 324 located in the inlet section 322 and extends toward the cylinder body 31. The hollow tube 324 is in communication with the inlet section 322 and the first intake port 325. A bottom side of the hollow tube 324 abuts the intake check valve 332. The inlet opening includes a first rib 313 extending in a diametric direction 312. The first rib 313 is spaced from the intake check valve 332 by a first spacing 314, providing room for the intake check valve 332. Thus, a free end of the intake check valve 332 is capable of swinging toward the cylinder body 31 to open the first intake passage such that an intake passage is provided when the piston 34 reciprocates. The outlet opening 315 includes a second rib 316 extending in a diametric direction. The second rib 316 abuts the outtake check valve 333. The outtake check valve 333 is spaced from the outlet section 323 by a second spacing 317, providing room for the outtake check valve 333. Thus, a free end of the outtake check valve 333 is capable of swinging toward the cover 32 to open the first outtake passage such that an outtake passage is provided when the piston 34 reciprocates. The second cylinder unit 4 is identical to the first cylinder unit 3. This is the preferred embodiment of the present invention. By the room design through the arrangement of the intake check valve 332 and the outtake check valve 333 of the spacer plate 33, the cover 32, and the cylinder body 31, the first and second cylinders 3 and 4 include the first intake port 325, the first outtake port 326, the second intake port 421, and the second outtake port 422.

In view of the foregoing, the present invention indeed meets industrial utility and has not been published or used in public and is not well known by the public. Furthermore, the present invention possess non-obviousness and meets the requirements of patentability. An application for patent is, thus, filed.

However, the above description merely sets forth a preferred embodiment of the present invention in the industry. Equivalent modifications to the claims of the present invention still fall within the scope of the present invention.

## Claims

1. An atomizer using a plurality of cylinder units to separate an inlet from an outlet, **characterized by** comprising:
a housing including an inlet and an outlet;
a motor mounted in the housing, with the motor including an eccentric transmission shaft;
a first cylinder unit mounted in the housing, with the first cylinder unit including a piston and a coupling bearing connected to the piston, with the eccentric transmission shaft extending through the coupling bearing, with the piston moving reciprocatingly when the motor operates, with the first cylinder unit further including a first intake port, with an intake check valve mounted in a first intake passage for the first intake port, with the first intake port connected to an end of a connection pipe, with the other end of the connection pipe connected to the inlet, forming a first intake piping; and
a second cylinder unit mounted in the housing, with the second cylinder unit including a piston and a coupling bearing connected to the piston, with the eccentric transmission shaft extending through the coupling bearing, with the piston moving reciprocatingly when the motor operates, with the second cylinder unit further including a second outtake port, with an outlet check valve mounted in a second outtake passage for the second outtake port, with the second outtake port connected to an end of a connection pipe, with the other end of the connection pipe connected to the outlet, forming a second outtake piping.

2. The atomizer using a plurality of cylinder units to separate an inlet from an outlet as claimed in claim 1, **characterized by** the first cylinder unit further including a first outtake port, with an outtake check valve mounted in a first outtake passage for the first outtake port, with the first outtake port connected to an end of a connection pipe, with the other end of the connection pipe connected to a first vent of the atomizer, forming a first outtake piping, with the second cylinder unit further including a second intake port, with an inlet check valve mounted in a second intake passage for the second intake port, with the second intake port connected to an end of a connection pipe, with the other end of the connection pipe connected to a second vent of the atomizer, forming a second intake piping.

3. The atomizer using a plurality of cylinder units to separate an inlet from an outlet as claimed in claim 1, **characterized by** the motor further including a mounting frame fixed to a side thereof, with the first and second cylinder units mounted to the mounting frame, with the eccentric transmission shaft located between the first and second cylinder units, and with the first and second cylinder units spaced from each other by 180 degrees as viewed from a longitudinal axis of the eccentric transmission shaft.

4. The atomizer using a plurality of cylinder units to separate an inlet from an outlet as claimed in claim 2, **characterized by** each of the first and second cylinder units including a cylinder body and a cover covering the cylinder body, with the first intake port and the first outtake port located in the cover of the first cylinder unit, and with the second intake port and the second outtake port located in the cover of the second cylinder unit.

5. The atomizer using a plurality of cylinder units to separate an inlet from an outlet as claimed in claim 4, **characterized by** the first cylinder unit further including a spacer plate between the cylinder body and the cover of the first cylinder unit, with a top wall formed on a side of the cylinder body unit facing the cover, with the top wall including an inlet opening and an outlet opening, with a partitioning wall extending from an upper wall of the cover toward the cylinder body, with a bottom face of the partitioning wall abutting the spacer plate, with the upper wall of the cover, the partitioning wall, and the spacer plate dividing an interior of the cover into an inlet section and an outlet section not in communication with the inlet section, with the inlet opening being in communication with the inlet section and the first intake port to form the first intake passage, with the outlet opening being in communication with the outlet section and the first outtake port to form the first outtake passage, with the spacer plate including two punched holes respectively aligned with the inlet opening and the outlet opening, with remaining materials in the two punched holes connected to the spacer plate and respectively forming the intake check valve and the outtake check valve, with the cover including a hollow tube located in the inlet section and extending toward the cylinder body, with the hollow tube being in communication with the inlet section and the intake port of the first cylinder unit, with a bottom side of the hollow tube abutting the intake check valve, with the inlet opening including a first rib extending in a diametric direction, with the first rib spaced from the intake check valve by a first spacing, providing room for the intake check valve, with a free end of the intake check valve capable of swinging toward the cylinder body to open the first intake passage, with the outlet opening including a second rib extending in a diametric direction, with the second rib abutting the outtake check valve, with the outtake check valve spaced from the outlet section by a second spacing, providing room for the outtake check valve, with a free end of the outtake check valve of the first cylinder unit having a free end capable of swinging toward the cover to open the first outtake passage.
